# EUROPEAN PATENT SPECIFICATION

(11) **EP 0 432 797 B1**
(45) Date of publication and mention of the grant of the patent: **22.06.1994**
(21) Application number: 90124210.7
(22) Date of filing: 14.12.1990
(51) Int. Cl.: C07C 61/24, C07C 51/083, C07C 51/493, C07C 51/48

(54) **Process for purification of 3,4,5,6-tetrahydrophthalic anhydride**
Verfahren zur Reinigung von 3,4,5,6-Tetrahydrophthalsäureanhydrid
Procédé de purification d'anhydride 3,4,5,6-tetrahydrophtalique

(30) Priority: 15.12.1989 JP 326698/89
(43) Date of publication of application: 19.06.1991
(73) Proprietor: NEW JAPAN CHEMICAL CO.,LTD., Kyoto-shi Kyoto-fu (JP)
(72) Inventor: Tani, Shoji, Nishinomiya-shi, Hyogo-ken (JP); Nishio, Kenji, Ootsu-shi, Shiga-ken (JP)
(74) Representative: Bohnenberger, Johannes, Dr.

(56) References cited:
- DE-A- 2 948 414
- CHEMICAL ABSTRACTS, vol. 83, no. 21, 24 November 1975, Columbus,Ohio, US; abstract no. 178432U, SHIGEO KAMIMURA ET AL.: 'Refiningtetrahydrophthalic anhydride' page 518 ; & JP-A-75 13790(MITSUBISHI CHEMICAL) 22 May 1975
- JOURNAL OF THE AMERICAN CHEMICAL SOCIETY. vol. 78, no. 15, 6 August 1956,GASTON, PA US pages 3828 - 3830; M. E. BAILEY ET AL.: 'Rearrangement of the Double Bond in cis-4-Cylohexene-1,2-dicarboxylic Anhydride'

## Description

The present invention relates to a process for highly purifying a 3,4,5,6-tetrahydrophthalic anhydride (hereinafter referred to as "Δ¹-THPA") which is an industrial material useful as an intermediate for preparation of insecticides, herbicides or the like.

We have proposed a method for preparing Δ¹-THPA in which a 1,2,3,6-tetrahydrophthalic anhydride (hereinafter referred to as "Δ⁴-THPA") is isomerized (Japanese Examined Patent Publications Nos. 54144/1983 and 54148/1983). These methods, although commercially advantageous, give a crude Δ¹-THPA as a reaction product which contains about 1 to about 15% of Δ⁴-THPA or like structural isomers of Δ¹-THPA, hexahydrophthalic anhydride, phthalic anhydride, etc. (hereinafter generally referred to as "contaminating phthalic anhydrides"). It is very difficult to separate these contaminating phthalic anhydrides from Δ¹-THPA because of similarities therebetween in the boiling point, solubility in solvents and like physical properties. A high-purity Δ¹-THPA has not been efficiently produced on industrial basis by usual distillation, usual recrystallization or like purification procedures.

An object of the present invention is to provide a process for producing a highly purified Δ¹-THPA on industrial basis from an acid anhydride mixture containing Δ¹-THPA as the main component.

We conducted extensive research on the solubility of contaminating phthalic anhydrides in solvents, and the reactivity thereof with various compounds, wherein the contaminating phthalic anhydrides include unreacted Δ⁴-THPA and phthalic anhydride, hexahydrophthalic anhydride which are produced as by-products in the preparation of Δ¹-THPA by isomerization of Δ⁴-THPA. Our research revealed the following. The contaminating phthalic anhydrides are more easily hydrolyzed or converted into monoesters than Δ¹-THPA; the dicarboxylic acids obtained by the hydrolysis and the carboxyl-containing monoesters of carboxylic acids produced by the monoesterification are more soluble in water and/or an alcohol solvent than Δ¹-THPA; and Δ¹-THPA undergoes substantially no chemical change upon contact with water and/or an alcohol solvent. The present invention has been accomplished based on these novel findings.

According to the present invention, there is provided a process for purifying 3,4,5,6-tetrahydrophthalic anhydride, the process comprising the steps of bringing a crude 3,4,5,6-tetrahydrophthalic anhydride (comprising 3,4,5,6-tetrahydrophthalic anhydride as the main component and an acid anhydride (a) as impurities which contains at least one of 1,2,3,6-tetrahydrophthalic anhydride, hexahydrophthalic anhydride, phthalic anhydride, and structural isomers of 3,4,5,6-tetrahydrophthalic anhydrides) into contact with a medium comprising at least one of water and monohydric or polyhydric alcohols at a temperature in the range of room temperature to 160°C so as to hydrolyze the acid anhydride (a) and/or convert it into the corresponding monoester; and separating the 3,4,5,6-tetrahydrophthalic anhydride in a solid state from the reaction mixture, the monohydric or polyhydric alcohols being selected from (i) an alcohol represented by the formula

ROH (A)

wherein R is an alkyl group having 1 to 22 carbon atoms, an alkenyl group having 3 to 18 carbon atoms, an aryl group, an aralkyl group or an alicyclic hydrocarbon group, (ii) an alcohol represented by the formula

HO-R¹-OH (B)

wherein R¹ is a group 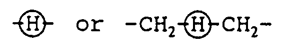
(wherein Ⓗ is an alicyclic hydrocarbon group having 6 carbon atoms), a group 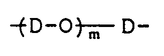
(wherein D is an alkylene group having 2 or 3 carbon atoms, and m is an integer of 0, 1 or 2), (iii) an alcohol represented by the formula 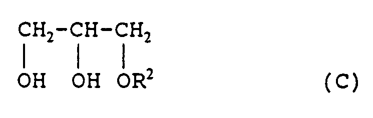
wherein R² is a hydrogen atom or a group 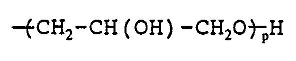
(wherein p is an integer of 1 or 2), and (iv) a mixture of such alcohols (i) to (iii).

To be brief, the present invention has the feature that a crude Δ¹-THPA is purified by contacting the crude Δ¹-THPA as the starting material with water and/or said alcohol to convert the contaminating phthalic anhydrides into the corresponding dicarboxylic acids and/or monoesters and separating Δ¹-THPA therefrom.

In other words, the process of the invention is characterized in that a crude Δ¹-THPA is purified by converting the contaminating acid anhydrides in the crude Δ¹-THPA into the corresponding dicarboxylic acids or monoesters which are soluble in water and/or the foregoing alcohol solvent, and removing the dicarboxylic acids and monoesters as dissolved in the solvent.

The amount of the contaminating phthalic anhydrides to be acceptable in the crude Δ¹-THPA as the starting material is not specifically limited insofar as the intended effect is obtained. Usually the amount is about 1 to about 20% by weight, preferably about 3 to about 15% by weight.

The kind of alcohols to be used in the invention are capable of forming the corresponding monoester by the reaction with the contaminating phthalic anhydrides and are represented by the formula (A), (B) or (C). These alcohols are usable singly, or at least two of them can be used in mixture.

The monohydric or polyhydric alcohols include (i) an alcohol represented by the formula

ROH (A)

wherein R is an alkyl group having 1 to 22 carbon atoms, an alkenyl group having 3 to 18 carbon atoms, an aryl group, an aralkyl group or an alicyclic hydrocarbon group, (ii) an alcohol represented by the formula

HO-R¹-OH (B)

wherein R¹ is a group 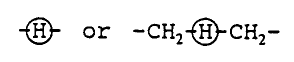
(wherein Ⓗ is an alicyclic hydrocarbon group having 6 carbon atoms), a group 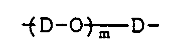
(wherein D is an alkylene group having 2 or 3 carbon atoms, and m is an integer of 0, 1 or 2), (iii) an alcohol represented by the formula 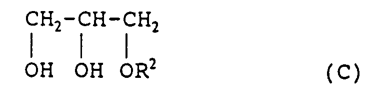
wherein R² is a hydrogen atom or a group 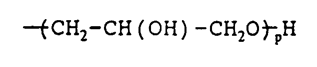
(wherein p is an integer of 1 or 2), and (iv) a mixture of such alcohols (i) to (iii).

Examples of the alcohols of the formula (A) are as follows:
(1) straight- or branched-chain aliphatic saturated or unsaturated monohydric alcohols having 1 to 22 carbon atoms, particularly aliphatic C₁-C₂₂ saturated or C₃-C₁₈ unsaturated monohydric alcohols, such as those containing groups including methyl, ethyl, propyl, butyl, isobutyl, amyl, hexyl, heptyl, octyl, 2-ethylhexyl, isooctyl, isononyl, decyl, isodecyl, undecyl, dodecyl, tridecyl, tetradecyl, hexadecyl, octadecyl, docosyl, allyl, octadecenyl, etc.;
(2) benzyl alcohol, phenethyl alcohol and like aralkyl alcohols; and
(3) cyclopentyl alcohol, cyclohexyl alcohol and like alicyclic monohydric alcohols.

Examples of the alcohols of the formula (B) are cyclohexanediol, cyclohexanedimethanol, ethylene glycol, diethylene glycol, triethylene glycol and like polyethyelene glycols, propylene glycol, dipropylene glycol, tripropylene glycol and like polypropylene glycols.

Examples of the alcohols of the formula (C) are aliphatic polyhydric alcohols such as glycerin, diglycerin, triglycerin and like polyglycerins.

The water and/or the alcohol may be used in an amount effective to hydrolyze the contaminating phthalic anhydrides or to convert the contaminating phthalic anhydrides into the monoesters thereof. The amount is usually 0.1 to 50 moles, preferably 0.5 to 20 moles, per mole of the crude Δ¹-THPA (in terms of the mean molecular weight as calculated from the neutralization value).

The process of the invention can be variously conducted. According to one preferred embodiment of the invention, a crude Δ¹-THPA and the above-specified amount of the solvent are mixed together and stirred with heating to dissolve the crude Δ¹-THPA in the solvent and to thereby effect the reaction and the reaction mixture is cooled, and the precipitated crystals are separated and dried under reduced pressure (hereinafter referred to as "method 1").

The heating in the method 1 is usually conducted at a temperature of 50 to 160°C, preferably 60 to 140°C, more preferably 70 to 140°C. If a temperature higher than 160°C is employed, the monoester is further esterified into a diester which is less soluble in water and/or alcohol solvents, and which consequently remains as mixed with Δ¹-THPA. If a temperature lower than 50°C is employed, the hydrolysis of contaminating phthalic anhydrides or the reaction thereof for conversion into monoesters slowly proceeds.

When the crude Δ¹-THPA is brought into contact with the water and/or the alcohol solvent at a temperature of 50 to 160°C, substantially the entire amount of the contaminating phthalic anhydrides is hydrolyzed and/or converted into monoesters in about 5 to about 60 minutes, and the thus produced dicarboxylic acids and/or monoesters are dissolved in the water and/or the alcohol solvent. The Δ¹-THPA in the crude Δ¹-THPA is dissolved in the water and/or the alcohol solvent without chemical change. When the reaction mixture is subsequently cooled to a temperature of, e.g. 10 to 50 °C, the dicarboxylic acids and/or monoesters remain dissolved in the solvent while the Δ¹-THPA is precipitated as crystals. The crystals are separated by usual separating means such as filtration or the like and dried under reduced pressure, giving the desired highly purified Δ¹-THPA.

According to another preferred embodiment of the invention, the process of the invention can be effected as follows. A crude Δ¹-THPA is mixed with the above-specified amount of water and/or an alcohol solvent with stirring for a period of time and at a temperature such that the Δ¹-THPA is not dissolved. Subsequently the reaction mixture is filtered, giving a highly purified Δ¹-THPA. This method is hereinafter referred to as "method 2".

The temperature to be employed in the method 2 is in the range of room temperature to 70°C, preferably 40 to 60°C, and the period of time may range from 10 to 120 minutes under this temperature condition. This method 2 is characterized in that substantially all the Δ¹-THPA in the crude Δ¹-THPA remains as a solid and the contaminating phthalic anhydrides alone are hydrolyzed or converted into their monoesters. At a higher temperature than 70°C which exceeds the melting point of Δ¹-THPA, the Δ¹-THPA can not be retained in a solid state. On the other hand, at a temperature lower than room temperature, it is difficult to hydrolyze the contaminating phthalic anhydrides as impurities or to convert them into monoesters.

In the purification of method 2, a crude Δ¹-THPA in the form of granules or powder is preferably used to efficiently remove the contaminating phthalic anhydrides as impurities.

When stirred in water and/or alcohol solvent under the foregoing temperature conditions for about 10 to about 120 minutes such that the Δ¹-THPA can be retained in a solid state, the contaminating phthalic anhydrides present as impurities in the solid crude Δ¹-THPA are hydrolyzed or converted into monoesters, and the resulting hydrolyzates and monoesters are dissolved in water and/or alcohol solvent. In the meantime, substantially the whole of Δ¹-THPA remains as a solid without undergoing dissolution nor chemical change. Subsequently the reaction mixture is cooled to approximately room temperature when so required and filtered, and the obtained Δ¹-THPA is dried at atmospheric or reduced pressure, whereby a highly purified Δ¹-THPA can be obtained in a high yield.

To improve the intended effect of the invention, an organic solvent which is unreactive with acid anhydrides can be used if so desired in the method 1 and method 2. Stated more specifically, the yield of Δ¹-THPA can be increased by use of such organic solvent which enables control of the solubility of dicarboxylic acids produced by the hydrolysis and/or monoesters of carboxylic acids produced by the monoesterification. Especially, when the water and/or alcohol is used in a small amount, the use of the organic solvent can increase the solubility of the dicarboxylic acids and/or monoesters.

Examples of useful organic solvents which are unreactive with acid anydrides are given below in (I) to (IV). They are usable singly, or at least two of them can be used in mixture.
(I) Hydrocarbons having 6 to 16 carbon atoms such as aliphatic hydrocarbons, alicyclic hydrocarbons and aromatic hydrocarbons, such as hexane, heptane, octane, decane, dodecane, cyclohexane, decalin, tetralin, toluene, xylene, ethyl benzene, butyl benzene, and mixed hydrocarbons produced in petroleum refining.
(II) Chain or cyclic ethers such as diethyl ether, diisopropyl ether, dibutyl ether, tetrahydrofuran, dioxane, etc.
(III) Chain or cyclic ketones such as acetone, methyl ethyl ketone, diethyl ketone, methyl isobutyl ketone, cyclopentanone, cyclohexanone, etc.
(IV) Other unreactive organic solvents such as acetonitrile and like nitriles, ethyl acetate, propyl acetate, butyl acetate and like esters, ethyl cellosolve, butyl cellosolve, and like polyethers.

The amount of the unreactive organic solvent to be used is not specifically limited. Yet the amount should not be excessive since an excessive amount tends to decrease the amount of obtained high-purity Δ¹-THPA. Thus the amount is usually 0 to 500% by weight, preferably 0 to about 200% by weight, based on the crude Δ¹-THPA.

When required, the Δ¹-THPA in the reaction system can be decolorized using active carbon or like adsorbent in the method 1. This step renders Δ¹-THPA almost colorless. Useful active carbon can be any of those commonly used. Active carbon is added in an amount of about 0.2 to about 10% by weight based on the weight of the starting crude Δ¹-THPA. After dissolution of the starting materials in the solvent, the decolorization can be easily done by stirring the solution for about 10 to about 120 minutes and filtering the reaction mixture. Other adsorbents such as activated clay and acid clay are also as effective as active carbon.

The present invention will be described below in greater detail with reference to the following examples and comparison examples.

### Example 1

A 1-liter four-necked flask equipped with a stirrer, a condenser, a thermometer and a nitrogen gas inlet was charged under nitrogen atmosphere with 300 g of isopropyl alcohol and 152 g of a crude Δ¹-THPA consisting of 90% by weight of Δ¹-THPA, 3% by weight of hexahydrophthalic anhydride, 1% by weight of phthalic anhydride, 2% by weight of a structural isomer of Δ¹-THPA and 4% by weight of other components (as assayed by GC-12A, trademark for a gas chromatography manufactured by Shimadzu Corp., the same hereinafter). The crude Δ¹-THPA was dissolved in the solvent with stirring and heating. The solution was stirred at 80°C for 30 minutes and then cooled to 25°C. The solution was filtered and dried under reduced pressure at 60°C at a pressure of 5 to 6 mmHg until no weight change occurred. The above procedure gave 132 g of Δ¹-THPA having a purity of 99.9% by weight in a yield of 97%.

### Examples 2 to 6

The same procedure as in Example 1 was conducted except that the amount of crude Δ¹-THPA, the kind of the solvent and the temperature were varied as shown in Table 1. Table 1 below shows the results.

### Example 7

The same procedure as in Example 1 was repeated although in a manner different in the following. To 300 g of isopropyl alcohol was added 152 g of powdery crude Δ¹-THPA consisting of 90% by weight of Δ¹-THPA, 3% by weight of hexahydrophthalic anhydride, 1% by weight of phthalic anhydride, 2% by weight of a structural isomer of Δ¹-THPA and 4% by weight of other components. The mixture was heated to 60°C and stirred for 10 minutes. The reaction product was filtered after cooling. The results are shown in Table 1.

### Example 8

The same procedure as in Example 7 was conducted with the exception of using 150 g of water and 150 g of isopropyl alcohol. Table 1 shows the results.

### Comparison Examples 1 and 2

The same procedure as in Example 1 was followed with the exception of using a solvent which is unreactive with acid anhydrides. Table 1 shows the results.

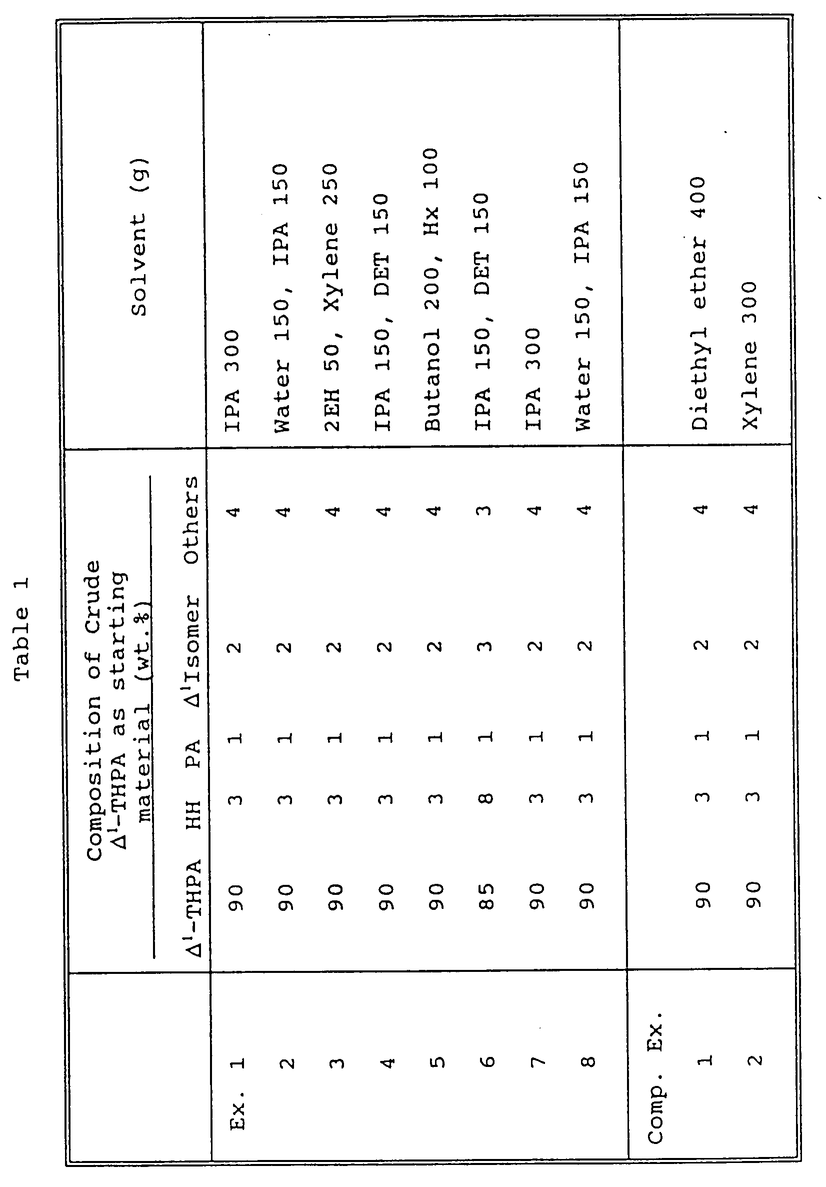

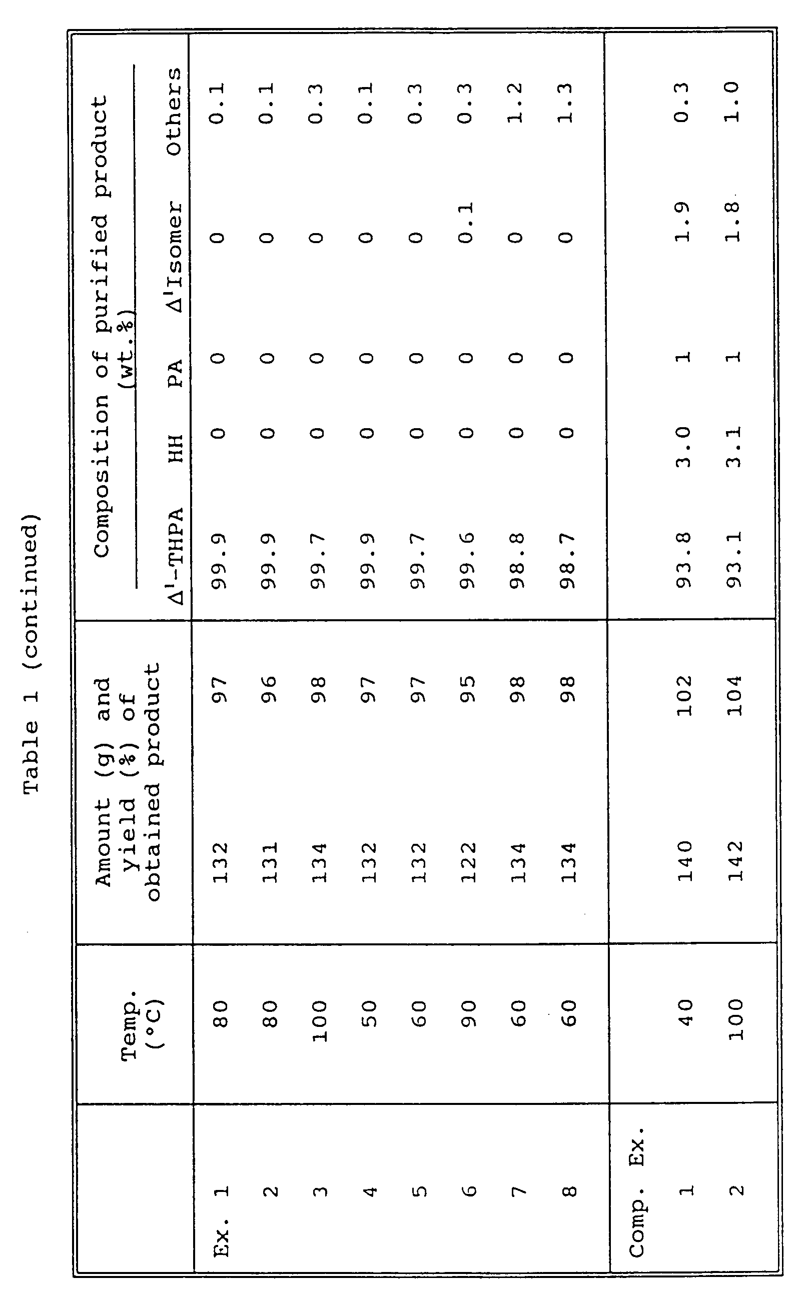

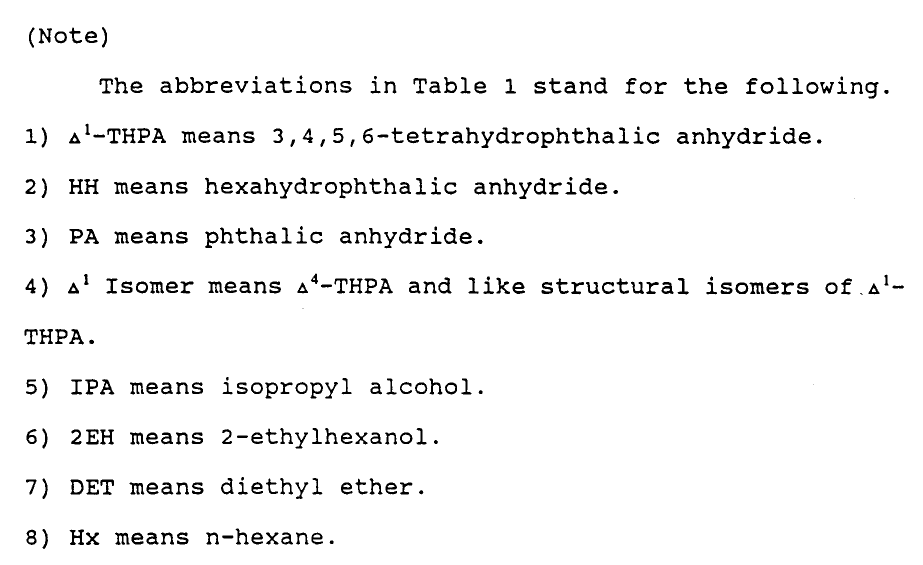

## Claims

1. A process for purifying 3,4,5,6-tetrahydrophthalic anhydride, the process comprising the steps of bringing a crude 3,4,5,6-tetrahydrophthalic anhydride (comprising a 3,4,5,6-tetrahydrophthalic anhydride as the main component and an acid anhydride (a) as impurities which contains at least one of 1,2,3,6-tetrahydrophthalic anhydride, hexahydrophthalic anhydride, phthalic anhydride, and structural isomers of 3,4,5,6-tetrahydrophthalic anhydride) into contact with a medium comprising at least one of water and monohydric or polyhydric alcohols at a temperature in the range of room temperature to 160°C so as to hydrolyze the acid anhydride (a) and/or convert it into a monoester; and separating the 3,4,5,6-tetrahydrophthalic anhydride in a solid state from the reaction mixture, the monohydric or polyhydric alcohols being selected from (i) an alcohol represented by the formula
ROH (A)
wherein R is an alkyl group having 1 to 22 carbon atoms, an alkenyl group having 3 to 18 carbon atoms, an aryl group, an aralkyl group or an alicyclic hydrocarbon group, (ii) an alcohol represented by the formula
HO-R¹-OH (B)
wherein R¹ is a group 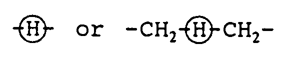 (wherein Ⓗ is an alicyclic hydrocarbon group having 6 carbon atoms), a group 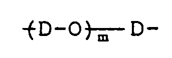 (wherein D is an alkylene group having 2 or 3 carbon atoms, and m is an integer of 0, 1 or 2), (iii) an alcohol represented by the formula 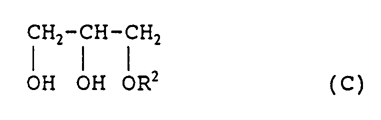 wherein R² is a hydrogen atom or a group 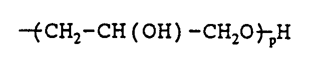 (wherein p is an integer of 1 or 2), and (iv) a mixture of such alcohols (i) to (iii).

2. A process according to claim 1 wherein the monohydric or polyhydric alcohol is at least one alcohol selected from the group consisting of straight- or branched-chain aliphatic saturated or unsaturated monohydric alcohols having 1 to 22 carbon atoms, benzyl alcohol, phenethyl alcohol, cyclopentyl alcohol, cyclohexyl alcohol, cyclohexanediol, cyclohexanedimethanol, ethylene glycol, diethylene glycol, triethylene glycol, propylene glycol, dipropylene glycol, tripropylene glycol, glycerin, diglycerin and triglycerin.

3. A process according to claim 1 or 2, wherein 0.1 to 50 moles of the water and/or the alcohol is used per mole of the crude 3,4,5,6-tetrahydrophthalic anhydride in terms of the mean molecular weight as calculated from the neutralization value.

4. A process according to claim 1 or 2, wherein 0.5 to 20 moles of the water and/or the alcohol is used per mole of the crude 3,4,5,6-tetrahydrophthalic anhydride in terms of the mean molecular weight as calculated from the neutralization value.

5. A process according to any one of claims 1 to 4 which comprises the steps of bringing the crude 3,4,5,6-tetrahydrophthalic anhydride into contact with the medium comprising at least one of water and monohydric or polyhydric alcohols at a temperature of 50 to 160°C so as to hydrolyze the acid anhydride (a) and/or convert it into a monoester, dissolving the obtained hydrolyzate and/or the monoester and the 3,4,5,6-tetrahydrophthalic anhydride in the solvent, cooling the solution to precipitate only the 3,4,5,6-tetrahydrophthalic anhydride as crystals, and separating the crystals from the solution.

6. A process according to any one of claims 1 to 4 which comprises the steps of bringing the crude 3,4,5,6-tetrahydrophthalic anhydride into contact with the medium comprising at least one of water and monohydric or polyhydric alcohols at a temperature of room temperature to 70°C so as to hydrolyze the acid anhydride (a) and/or convert it into a monoester, dissolving the obtained hydrolyzate and/or the monoester in the solvent, and separating the 3,4,5,6-tetrahydrophthalic anhydride remaining as a solid.

7. A process according to any one of claims 1 to 6 wherein the medium further contains an organic solvent which is unreactive with acid anhydrides.

8. A process according to claim 7 wherein the organic solvent is at least one solvent selected from the group consisting of hydrocarbons having 6 to 16 carbon atoms, chain or cyclic ethers, chain or cyclic ketones, nitriles, esters and polyethers.

9. A process according to claim 7 or 8 wherein the organic solvent is used in an amount of 500% by weight or less based on the crude 3,4,5,6-tetrahydrophthalic anhydride.

## Patentansprüche

1. Verfahren zum Reinigen von 3,4,5,6-Tetrahydrophthalsäureanhydrid, wobei das Verfahren folgende Schritte aufweist: In-Kontakt-Bringen von rohem 3,4,5,6-Tetrahydrophthalsäureanhydrid (das ein 3,4,5,6-Tetrahydrophthalsäureanhydrid als die Hauptkomponente und ein Säureanhydrid (a) als Verunreinigungen, das mindestens eines von 1,2,3,6-Tetrahydrophthalsäureanhydrid, Hexahydrophthalsäureanhydrid, Phthalsäureanhydrid und Strukturisomeren von 3,4,5,6-Tetrahydrophthalsäureanhydrid enthält, aufweist) mit einem Medium, das mindestens entweder Wasser oder ein- oder mehrwertige Alkohole aufweist, bei einer Temperatur im Bereich zwischen Raumtemperatur und 160 °C, um das Säureanhydrid (a) zu hydrolysieren und/oder zu einem Monoester umzusetzen; und Abtrennen des 3,4,5,6-Tetrahydrophthalsäureanhydrids in einem festen Zustand aus dem Reaktionsgemisch, wobei die ein- oder mehrwertigen Alkohole ausgewählt sind aus (i) einem Alkohol, der dargestellt ist durch die Formel
ROH (A)
in der R eine Alkylgruppe mit 1-22 Kohlenstoffatomen, eine Alkenylgruppe mit 3-18 Kohlenstoffatomen, eine Arylgruppe, eine Aralkylgruppe oder eine alicyclische Kohlenwasserstoffgruppe ist, (ii) einem Alkohol, der dargestellt ist durch die Formel
HO-R¹-OH (B)
in der R¹ eine Gruppe 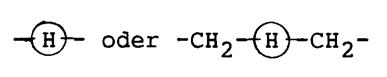 (mit Ⓗ = eine alicylische Kohlenwasserstoffgruppe mit 6 Kohlenstoffatomen), eine Gruppe 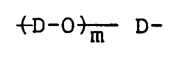 (mit D = eine Alkylengruppe mit 2 oder 3 Kohlenstoffatomen und m = eine ganze Zahl von 0, 1 oder 2) ist, (iii) einem Alkohol, der dargestellt ist durch die Formel 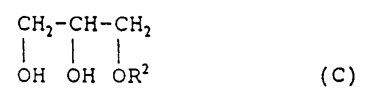 in der R² ein Wasserstoffatom oder eine Gruppe 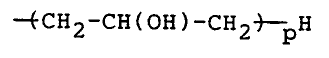 (mit p = eine ganze Zahl von 1 oder 2) ist, und (iv) einem Gemisch von solchen Alkoholen (i) bis (iii).

2. Verfahren nach Anspruch 1, wobei der ein- oder mehrwertige Alkohol mindestens ein Alkohol ist, der aus der Gruppe ausgewählt ist, die aus folgenden besteht: gerad- oder verzweigtkettigen aliphatischen gesättigten oder ungesättigten einwertigen Alkoholen mit 1-22 Kohlenstoffatomen, Benzyl-, Phenethyl-, Cyclopentyl-, Cyclohexylalkohol, Cyclohexandiol, Cyclohexandimethanol, Ethylen-, Diethylen-, Triethylen-, Propylen-, Dipropylen-, Tripropylenglycol, Glycerin, Diglycerin und Triglycerin.

3. Verfahren nach Anspruch 1 oder 2, wobei 0,1 bis 50 mol des Wassers und/oder des Alkohols pro mol des rohen 3,4,5,6-Tetrahydrophthalsäureanhydrids, ausgedrückt als das aus der Neutralisationszahl berechnete mittlere Molekulargewicht, eingesetzt werden.

4. Verfahren nach Anspruch 1 oder 2, wobei 0,5 bis 20 mol des Wassers und/oder des Alkohols pro mol des rohen 3,4,5,6-Tetrahydrophthalsäureanhydrids, ausgedrückt als das aus der Neutralisationszahl berechnete mittlere Molekulargewicht, eingesetzt werden.

5. Verfahren nach einem der Ansprüche 1-4, das die folgenden Schritte aufweist: In-Kontakt-Bringen des rohen 3,4,5,6-Tetrahydrophthalsäureanhydrids mit dem Medium, das mindestens entweder Wasser oder ein- oder mehrwertige Alkohole aufweist, bei einer Temperatur von 50-160 °C, um das Säureanhydrid (a) zu hydrolysieren und/oder zu einem Monoester umzusetzen, Lösen des erhaltenen Hydrolysats und/oder des Monoesters und des 3,4,5,6-Tetrahydrophthalsäureanhydrids in dem Lösungsmittel, Abkühlen der Lösung, um nur das 3,4,5,6-Tetrahydrophthalsäureanhydrid als Kristalle auszufällen, und Trennen der Kristalle von der Lösung.

6. Verfahren nach einem der Ansprüche 1-4, das die folgenden Schritte aufweist: In-Kontakt-Bringen des rohen 3,4,5,6-Tetrahydrophthalsäureanhydrids mit dem Medium, das mindestens entweder Wasser oder ein- oder mehrwertige Alkohole aufweist, bei einer Temperatur zwischen Raumtemperatur und 70 °C, um das Säureanhydrid (a) zu hydrolysieren und/oder zu einem Monoester umzusetzen, Lösen des erhaltenen Hydrolysats und/oder des Monoesters in dem Lösungsmittel und Trennen des als ein Feststoff verbleibenden 3,4,5,6-Tetrahydrophthalsäureanhydrids.

7. Verfahren nach einem der Ansprüche 1-6, wobei das Medium ferner ein organisches Lösungsmittel enthält, das mit den Säureanhydriden nicht reagiert.

8. Verfahren nach Anspruch 7, wobei das organische Lösungsmittel mindestens ein Lösungsmittel ist, das aus der Gruppe ausgewählt ist, die aus folgenden besteht: Kohlenwasserstoffen mit 6-16 Kohlenstoffatomen, Ketten- oder Ringethern, Ketten- oder Ringketonen, Nitrilen, Estern und Polyethern.

9. Verfahren nach Anspruch 7 oder 8, wobei das organische Lösungsmittel in einer Menge von 500 Gew.-% oder weniger, bezogen auf das rohe 3,4,5,6-Tetrahydrophthalsäureanhydrid, eingesetzt wird.

## Revendications

1. Procédé pour purifier un anhydride 3,4,5,6-tétrahydrophtalique, le procédé comprenant les étapes suivantes : amener un anhydride 3,4,5,6-tétrahydrophtalique brut (comprenant un anhydride 3,4,5,6-tétrahydrophtalique comme composant principal et un anhydride d'acide (a) comme impuretés, qui contient au moins l'un d'un anhydride 1,2,3,6-tétrahydrophtalique, un anhydride hexahydrophtalique, un anhydride phtalique et des isomères structuraux d'anhydride 3,4,5,6-tétrahydrophtalique) en contact avec un milieu comprenant au moins l'un des composés suivants : eau et alcools monohydriques ou polyhydriques, à une température comprise entre la température ambiante et 160°C de façon à hydrolyser l'anhydride d'acide (a) et/ou le convertir en un monoester; et séparer du mélange de réaction l'anhydride 3,4,5,6-tétrahydrophtalique à l'état solide, les alcools monohydriques ou polyhydriques étant choisis parmi (i) un alcool représenté par la formule :
ROH (A)
dans laquelle R est un groupe alcoyle ayant 1 à 22 atomes de carbone, un groupe alcényle ayant 3 à 18 atomes de carbone, un groupe aryle, un groupe aralcoyle ou un groupe hydrocarbure alicyclique, (ii) un alcool représenté par la formule :
HO-R'-OH (B)
dans laquelle R' est un groupe 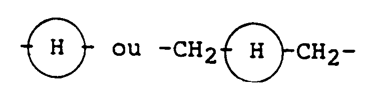 (dans laquelle Ⓗ est un groupe hydrocarbure alicyclique ayant 6 atomes de carbone), un groupe -(D-O)ₘ-D- (dans laquelle D est un groupe alcoylène ayant 2 à 3 atomes de carbone, et m est égal à 0, 1 ou 2), (iii) un alcool représenté par la formule : 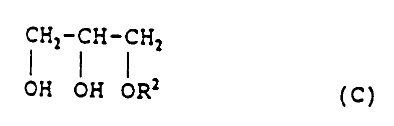 dans laquelle R2 est un atome d'hydrogène ou un groupe -(CH₂-CH(OH)-CH₂O)ₚ-H (dans laquelle p est égal à 1 ou 2), et (iv) un mélange des alcools (i) à (iii).

2. Procédé selon la revendication 1, dans lequel l'alcool monohydrique ou polyhydrique est au moins un alcool choisi dans le groupe comprenant les alcools suivants : alcool monohydrique, saturé ou insaturé, aliphatique à chaîne rectiligne ou ramifiée ayant 1 à 22 atomes de carbone, alcool benzylique, alcool phénéthylique, alcool cyclopentylique, alcool cyclohexylique, cyclohexanediol, cyclohexanediméthanol, éthylène glycol, diéthylène glycol, triéthylène glycol, propylène glycol, dipropylène glycol, tripropylène glycol, glycérine, diglycérine et triglycérine.

3. Procédé selon la revendication 1 ou la revendication 2, dans lequel on utilise 0,1 à 50 moles d'eau et/ou d'alcool par mole de l'anhydride 3,4,5,6-tétrahydrophtalique brut en termes de poids moléculaire moyen comme calculé à partir de la valeur de neutralisation.

4. Procédé selon la revendication 1 ou la revendication 2, dans lequel on utilise 0,5 à 20 moles d'eau et/ou d'alcool par mole de l'anhydride 3,4,5,6-tétrahydrophtalique brut en termes de poids moléculaire moyen comme calculé à partir de la valeur de neutralisation.

5. Procédé selon l'une des revendications 1 à 4, qui comprend les étapes suivantes : amener l'anhydride 3,4,5,6-tétrahydrophtalique brut en contact avec le milieu comprenant au moins de l'eau et/ou des alcools monohydriques ou polyhydriques, à une température de 50 à 160°C, de façon à hydrolyser l'anhydride d'acide (a) et/ou le convertir en un monoester, dissoudre l'hydrolysat et/ou le monoester obtenu et l'anhydride 3,4,5,6-tétrahydrophtalique dans le solvant, refroidir la solution pour précipiter en tant que cristaux seulement l'anhydride 3,4,5,6-tétrahydrophtalique, et séparer les cristaux de la solution.

6. Procédé selon l'une des revendications 1 à 4, qui comprend les étapes suivantes : amener l'anhydride 3,4,5,6-tétrahydrophtalique brut en contact avec le milieu comprenant au moins de l'eau et/ou des alcools monohydriques ou polyhydriques à une température comprise entre la température ambiante et 70°C de façon à hydrolyser l'anhydride d'acide (a) et/ou le convertir en un monoester, dissoudre l'hydrolysat et/ou le monoester obtenu dans le solvant, et séparer en tant que solide l'anhydride 3,4,5,6-tétrahydrophtalique résiduel.

7. Procédé selon l'une des revendications 1 à 6, dans lequel le milieu contient en outre un solvant organique qui ne réagit pas avec les anhydrides d'acide.

8. Procédé selon la revendication 7, dans lequel le solvant organique est au moins un solvant choisi dans le groupe comprenant des hydrocarbures ayant 6 à 16 atomes de carbone, des éthers en chaîne ou cycliques, des cétones en chaîne ou cycliques, des nitriles, des esters et des polyéthers.

9. Procédé selon la revendication 7 ou la revendication 8, dans lequel le solvant organique est utilisé en une quantité de 500% en poids ou moins sur la base de l'anhydride 3,4,5,6-tétrahydrophtalique brut.
